# EUROPEAN PATENT APPLICATION

(11) **EP 1 059 378 A1**
(43) Date of publication of application: **13.12.2000**
(21) Application number: 99111165.9
(22) Date of filing: 08.06.1999
(51) Int. Cl.: D04H 1/60, D04H 1/64

(54) **Wet wipe with antifoaming agent**

(71) Applicant: THE PROCTER & GAMBLE COMPANY, Cincinnati, Ohio 45202 (US)
(72) Inventor: Pucci, Maurizio, 44100 Ferrara (IT)
(74) Representative: Kohol, Sonia

(57) **Abstract**

The present invention relates to a process for the production of a web comprising a binder and an antifoaming agent. More particularly, the present invention relates to a process for the production of a web suitable for the provision of wet wipes. One main application of these wet wipes is in hard surface cleaning. Claimed and described is a process for the production of a web whereby the binder and the antifoaming agent are each cured after their respective application to the web.

## Description

### Field of the Invention

The present invention relates to a process for the production of a web comprising a binder and an antifoaming agent. More particularly, the present process relates to a process for the production of a web suitable for the provision of wet wipes. One main application of these wet wipes is in hard surface cleaning.

### Background of the Invention

Wet wipes are typically pre-moistened, disposable towelettes which may be utilised in a variety of applications both domestic and industrial and perform a variety of functions. Wet wipes are typically used to wipe surfaces both animate and inanimate, and may provide numerous benefits such as cleaning, cleansing, disinfecting, and skin care benefits.

One particular application for wet wipes is wiping and or cleaning surfaces and the application of compositions to surfaces, for example kitchen and bathroom surfaces, eyeglasses, shoes and surfaces which require cleaning in industry for example surfaces of machinery or automobiles. In addition wet wipes are also applicable for wiping parts of the human body particularly when wash water is not available, for example when travelling. Wipes are commonly used for human cleansing and wiping such as face and hand cleansing and anal, perineal and genital cleansing for example as intimate hygiene wipes such as feminine wet wipes. Wet wipes may also be used for application of substances to the body including removing and applying of make-up, skin conditioners and medications. Another application of wipes is during diaper changes and also for the treatment of adult and baby dermatitis partly caused by the use of diapers and incontinence devices. Wet wipes also include articles used for the cleaning or grooming of pets.

Wet wipes are commonly constructed from combinations of synthetic, man-made and natural fibres, such as polyolefin fibres, viscose fibres and cotton fibres, which are generally moistened with an aqueous composition which contains amongst others surfactants, preservatives, oils and scents. The wet wipes are then typically provided in a folded stacked configuration within a covered container such as a tub-like container having a lid. Alternatively, the wipes may be provided in tub containers having a dispensing aperture. In both cases this allows easy transportation and storage of the wipes. Typically the wipes are packaged in a plastic foil in order to protect the wet wipes prior to use.

A variety of webs, woven and non-woven, including those suitable for wet wipes, and processes for the production of such webs are known and disclosed in the art listed below. The addition of a binder is well known in the art to improve the strength of a web against tearing, in particular when wet. Foam reducing agents for various applications, including silicone based agents, are also well known. Moreover, various other silicon based agents are commonly added to wet wipes as to achieve various benefits.

WO 96/19616 discloses a tissue paper comprising a binder material and a polysiloxane compound, which is added for the softening of the paper. The binder, as common in the paper making art, is added at the wet end, i.e. to the paper making furnish. Only the polysiloxane softnener is later added onto the dried tissue paper web. The invention is said to be applicable to tissue paper in general, while non-woven materials are not mentioned.

For the area on non-woven webs, EP 0 333 209 discloses a method for producing a non-woven web with elastomeric properties, which is suitable for wipes. The method is said to improve the physical properties of the web with regard to strength and low linting and discloses the optional addition of a binder to the formed web, e.g. via an immersing bath.

EP 0 343 304 discloses meltblown wipes designed for the absorption of primarily water and oil. Effective absorption is said to be promoted by the addition of an organosilicone surfactant serving as a wetting agent. The mechanism of adding such wetting agent is not specified and said not to be critical to the invention.

EP 0 233 943 discloses a wet wipe comprising a binder and an antimicrobial agent. The binder and the antimicrobial agent are both applied to the unbonded, but formed, e.g. air-laid, web from which the wet wipes are provided. In one preferred method the web passes through a bath in which the antimicrobial agent and the binder a present. In a consecutive step the web comprising the antimicrobial agent and the binder is cured.

Some desirable liquid compositions for wet wipes, namely those comprising surfactants, are known to produce foam. Such foam may induce residues, which compromise the cleaning achieved by a wet wipe. High amounts of residues create a need to post-treat, typically rinse, the surfaces previously treated with the wet wipe. Such an additional post-treatment step makes a wet wipe less comfortable to use.

It is hence desirable to provide a wet wipe comprising an antifoaming agent. However, it has been found, that the application of an antifoaming agent when mixed with a binder, as disclosed in EP 0 233 943 for an antimicrobial agent, compromises the antifoaming efficiency of the agent. Therefore, it is further desirable to optimise the method of application of an antifoaming agent to a web.

It is yet further desirable to provide such antifoaming agent without compromising other properties of the wet wipe, namely the cleaning and disinfecting performance. It also is desirable to provide wet wipes which do not produce foam irrespective of the liquid compositions used. In addition, it remains desirable to provide a wet wipe, suitable for hard surface cleaning, which is strong, soft, absorbent and provides improved cleaning.

### Summary of the Invention

The present invention relates to a process for the production of a web comprising a binder and an antifoaming agent. More particularly, the present invention relates to a process for the production of a web suitable for the provision of wet wipes. One main application of these wet wipes is in hard surface cleaning. Claimed and described is a process for the production of a web whereby the binder and the antifoaming agent are each cured after their respective application to the web.

### Brief description of the figures

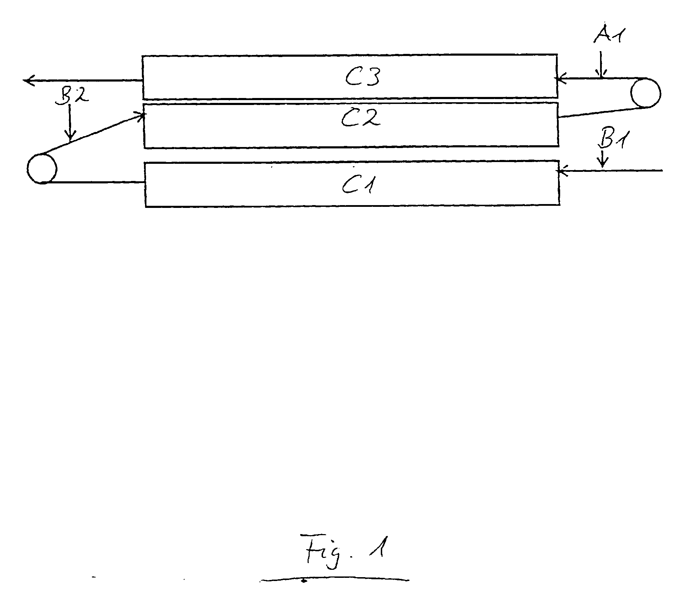

- Figure 1:: is a schematic view of a process according to the present invention. B1 and B2 denote binder application steps, C1 to C3 denote curing steps, and A1 denotes a step of application of an antifoaming agent

### Detailed Description of the Invention

The present invention relates to a process for producing a web. The process is applicable to any type of web, namely including wovens, non-wovens, paper tissues, whether made of natural, man-made or synthetic fibres, and other webs listed below.

To further illustrate the process, a process for producing a non-woven web suitable for wet wipes is discussed hereinafter. This process is selected only as an example and without any intention of limiting the claimed invention thereto.

Preferred wet wipes in accordance with the present invention are wet wipes for hard surface cleaning, other wet wipes to be produced by the present process encompass moist toilet paper, paper towels, facial tissues, baby wipes and other similar products. These wet wipes comprise a sheet of material provided from the web, a binder and an antifoaming agent, preferably also a liquid composition, all described hereinafter.

It has now been found that the process of application of the binder and the antifoaming agent is highly relevant to the performance of the wet wipes. According to the present invention the application of the binder to the web is followed by a step of curing the binder and also the application of the antifoaming agent is followed of a step of curing the antifoaming agent. The application and subsequent curing of the binder are herein referred to as binder treatment. The application and subsequent curing of the antifoaming agent are herein referred to as antifoaming treatment.

A process according to the present invention may comprise any number of binder treatments and any number of antifoaming treatments. Preferably all binder treatments are carried out prior to antifoaming treatments. A highly preferred process comprises two binder treatments and two antifoaming treatments. In this highly preferred process each of the two binder treatments and each of the two antifoaming treatments has an effect primarily on one side of the web.

A process for the production of a wet wipe in accordance with the present invention overall comprises the following steps: Firstly the process comprises initial steps for the production of a web, preferably a non-woven web by methods known in the art, such as hydroentangling, air-laying and others given below. Air-laying of non-woven web is preferred. Such a web will typically be supported by a belt.

On such a belt the web then passes a means for the application of a binder onto at least one side of the web, preferably a latex binder. While the binder can be applied to both sides of the web in one step, preferably the binder is applied to each side of the web in consecutive steps.

The binder can be applied to the web by any method known in the art. Suitable methods include spraying, printing (e.g. flexographic printing), coating (e.g. gravure coating or flood coating), padding, foaming, impregnation, saturation and further extrusion whereby the composition is forced through tubes in contact with the substrate whilst the substrate passes across the tube or combinations of these application techniques. For example spraying the composition on a rotating surface such as calendar roll that then transfers the composition to the surface of the substrate. The most preferred method for the application of the binder is spraying onto the web. Most preferably the binder is sprayed onto one side of the web in one step of application and onto the other side of the web in a independent step of application.

At least the last step of application of a binder is followed, preferably immediately followed, by at least one step of curing. A single curing step is preferred. Preferably each step of application of the binder is followed, preferably immediately followed, by a curing step. "Followed", as used herein, denotes that no other substance is applied to the web after a first treatment step, e.g. application of the binder, and before a second treatment step, e.g. curing. For a preferred process the application of the binder is immediately followed by a curing step. "Followed immediately", as used herein, denotes that the web is not subject to any other treatment but sheer moving, e.g. on a belt.

Suitable methods of curing are any methods which subject the web to heat. Such methods encompass the use of ovens, dryers, e.g. drum dryers, or steam cans. Preferred curing temperatures are 25°C to 500°C, more preferably from 100°C to 300°C, yet more preferably from 150°C to 250°C, most preferably from 180°C to 200 °C. For curing the web is subjected to heat for a span of time from 1 ps to 10 hours, more preferably from 1 ms to 1 hour, yet more preferably from Is to 10 minutes, most preferably from 1 s to 180 s.

The steps of binder treatment are preferably followed by steps of antifoaming treatment. Most preferably the steps of binder treatment are immediately followed by steps of antifoaming treatment. (An alternative process in which the antifoaming treatment is followed by the binder treatment is also within the scope of the present invention.) The antifoaming agent can be applied by any suitable method, namely any method listed for the binder. At least the last step of application of the antifoaming agent is followed, and preferably immediately followed, by at least one curing step. For a preferred method of the present invention each application of an antifoaming agent is followed, and preferably immediately followed, by one curing step.

The methods of curing after the application of the antifoaming agent are the same as performed after the application of the binder. In particular, the preferred curing temperature after application of the antifoaming agent is also in the range of 180°C to 200°C.

Figure 1 gives an example for a preferred process according to the present invention. In the depicted process the web is spray coated with a latex binder from one side (B1). The web then passes through an oven held at a temperature of 190°C (C1). The other side of the web is then spray coated with a latex binder (B 2). The web passes through a further oven held at 190°C (C 2). In a next step a silicone comprising antifoaming agent is sprayed onto the web (A 1). Thereafter is web passes through another oven held at 190°C (C 3).

While the following process steps, which are preferred but optional, may follow immediately, in a preferred process these steps follow only at a convenient later point in the time, e.g. a period of hours, days or weeks after the above described steps.

The web having received the described binder treatment and antifoaming treatment is impregnated with the liquid composition according to any means known in the art. The web is then cut in sheets of material of the desired size for the wet wipe. These sheets are typically folded, stacked and packaged, e.g. in a moisture proof plastic tub container with a lid.

It has been identified that wet wipes made according to the present process deposit less of the comprised antifoaming agent on treated surfaces than wet wipes comprising the same amount of antifoaming agent in the liquid composition which is added to the wipe. The deposition of the antifoaming agent on the treated surface is not desirable since such antifoaming agents typically leave residues which are directly visible or which at least make the treated surface less shiny. The effect is in particular known for silicone based antifoaming agents. Comparative data given below demonstrate a dependence of the shine of a treated surface and the production process of the wipes used for the treatment. Wet wipe made in accordance with the present invention lend more shine to the treated surfaces. At the same time the wet wipes do not deposit foam on treated surfaces.

Without wishing to be bound by theory it is believed that the application of an antifoaming agent to the web, if not followed by a curing step, leads to a considerable amount of mixing between the antifoaming agent and the liquid composition, which is typically added to the web after completion of the web making process. Hence when using a wipe made according to such a process the antifoaming agent is deposited onto a treated surface together with the liquid composition.

Again without wishing to be bound by theory it is believed that an alternative process in which binder and antifoaming agent are applied and subsequently both together are cured, leads to the polymerisation of the antifoaming agent in the binder, making the antifoaming agent considerably less effective.

### Web

The web comprises the sheets of material from which the wipes are produced. According to the present invention the web comprises a substrate which is preferably coated or impregnated with a liquid composition. The web may be woven or nonwoven, foam, sponge, battings, balls, puffs, films, or tissue paper, most preferably a nonwoven and may be composed or natural or synthetic fibres or mixtures thereof. Preferably, the fibre compositions are a mixed of hydrophilic fibre material such as viscose, cotton, or flax and a hydrophobic fibre material such as polyethylene tetraphthalate (PET) or polypropylene (PP) in a ratio of 10%-90% hydrophilic and 90%-10% hydrophobic material by weight. Particularly preferred compositions are 50% viscose, 50% PP; and 50% viscose, 50 % PET; and 70% cellulose, 15% PET and 15% latex. Another preferred composition is 70% cellulose, 15% man-made fibres and 15% latex. The web preferably has a basis weight of at least 20 gm⁻² and preferably less than 150 gm⁻², and most preferably the base weight is in the range of 20 gm⁻² to 100 gm⁻², more preferably from 50 gm⁻² to 95 gm⁻². The web may have any caliper. Typically, when the web is made by an air laying process, the average web caliper is less than 1.0 mm. More preferably the average caliper of the web is from 0.1 mm to 0.9 mm. The web caliper is measured according to standard EDANA nonwoven industry methodology, reference method # 30.4-89.

In addition to the fibres used to make the web, the web can have other components or materials added thereto as known in the art, for example agents to improve the optical characteristics of the material such as opacifying agents, for example titanium dioxide.

According to the present invention the sheet may be produced by any methods known in the art. For example nonwoven material substrates can be formed by dry forming techniques such as air-laying or wet laying such as on a paper making machine. Other nonwoven manufacturing techniques such as melt blown, spun bonded, needle punched and spun laced methods may also be used. A preferred method is air-laying.

### Binder

The web according to the present invention comprises a binder. The term "binder" as used herein describes any agent employed to interlock fibers. Such agents comprise wet strength resins and dry strength resins. It is often desirable particularly for cellulose based materials to add chemical substances known in the art as wet strength resins. A general dissertation on the types of wet strength resins utilised namely in the paper art can be found in TAPPI monograph series No. 29, Wet Strength in Paper and Paperboard, Technical Association of the Pulp and Paper Industry (New York, 1965). In addition to wet strength additives, it can also be desirable to include certain dry strength and lint control additives known in the art such as starch binders.

Preferred binders used to bond non-wovens are polymeric binders, preferably latex binders, more preferably waterborne latex binders. Suitable binders include, butadiene-styrene emulsions, ethylene vinyl acetate emulsions, vinyl acetate, vinyl chloride, and combinations thereof. Preferred latex binders are made from styrene, butadiene, acrylonitrile-butadiene emulsions or combination thereof.

According to the present invention non-acrylate binders are preferred. The term non-acrylate binder, as used herein, encompasses all latex binders that do not comprise acrylic acid or acrylic acid ester or vinyl acetate monomers. Preferred binders according to the present invention include: Butadiene-styrene emulsions, Carboxylated styrene-butadiene emulsion, Acrylonitrile-butadiene emulsions, Polyacrylamide resins, Polyamide-epichlorohydrin resin, Acrylonitrile-Butadiene-Styrene emulsion and Styrene Acrylonitrile. The most preferred binder according the present invention is a butadiene-styrene emulsion, which can be commercially obtained from the Ameribol Svnpol Corp. as Rowene™ SB 5550.

Typically, the amount of the binder applied to the web, as measured in weight % of the dry weight of the fibres comprised by the web, is from 5% to 30%, more preferably from 10% to 25 %, most preferably from 14% to 22%. Of course the amount of binder to be applied largely depends on the type to web to be treated.

### Antifoaming agent

The web according to the present invention comprises an antifoaming agent, herein also referred to as a foam reducing system. Any antifoaming agent known in the art is suitable for the present invention. Highly preferred antifoaming agents are those comprising silicone. Preferred antifoaming agents may further comprise a fatty acid together with a capped alkoxylated nonionic surfactant as defined herein after.

Preferably the amount of antifoaming agent used expressed in weight percent is from 0.01% to 5%, more preferably from 0.1% to 1%, most preferably from 0.1% to 0.6 % of the weight of the dry substrate material.

Suitable fatty acids for use herein are the alkali salts of a C₈-C₂₄ fatty acid. Such alkali salts include the metal fully saturated salts like sodium, potassium and/or lithium salts as well as the ammonium and/or alkylammonium salts of fatty acids, preferably the sodium salt. Preferred fatty acids for use herein contain from 8 to 22 carbon atoms, preferably from 8 to 20 and more preferably from 8 to 18.

Suitable fatty acids may be selected from caprylic acid, capric acid, lauric acid, myristic acid, palmitic acid, stearic acid and mixtures of fatty acids suitably hardened, derived from natural sources such as plant or animal esters (e.g., palm oil, coconut oil, soybean oil, castor oil, tallow, ground oil, whale and fish oils and/or babassu oil.

For example Coconut Fatty Acid is commercially available from UNICHEMA under the name PRIFAC 5900®.

Suitable capped alkoxylated nonionic surfactants for use herein are according to the formula:

R₁(O-CH₂-CH₂)ₙ-(OR₂)ₘ-O-R₃

wherein R₁ is a C₈-C₂₄ linear or branched alkyl or alkenyl group, aryl group, alkaryl group, preferably R₁ is a C₈-C₁₈ alkyl or alkenyl group, more preferably a C₁₀-C₁₅ alkyl or alkenyl group, even more preferably a C₁₀-C₁₅ alkyl group;
wherein R₂ is a C₁-C₁₀ linear or branched alkyl group, preferably a C₂-C₁₀ linear or branched alkyl group, preferably a C₃ group;
wherein R₃ is a C₁-C₁₀ alkyl or alkenyl group, preferably a C₁-C₅ alkyl group, more preferably methyl;
and wherein n and m are integers independently ranging in the range of from 1 to 20, preferably from 1 to 10, more preferably from 1 to 5; or mixtures thereof.

These surfactants are commercially available from BASF under the trade name Plurafac®, from HOECHST under the trade name Genapol® or from ICI under the trade name Symperonic®. Preferred capped nonionic alkoxylated surfactants of the above formula are those commercially available under the tradename Genapol® L 2.5 NR from Hoechst, and Plurafac® from BASF.

Suitable silicones for use herein include any silicone and silica-silicone mixtures. Silicones can be generally represented by alkylated polysiloxane materials while silica is normally used in finely divided forms exemplified by silica aerogels and xerogels and hydrophobic silicas of various types. These materials can be incorporated as particulates in which the silicone is advantageously releasably incorporated in a water-soluble or water-dispersible, substantially non-surface-active detergent impermeable carrier. Alternatively, the silicone can be dissolved or dispersed in a liquid carrier and applied by spraying on to one or more of the other components.

Actually in industrial practice, the term "silicone" has become a generic term which encompasses a variety of relatively high-molecular-weight polymers containing siloxane units and hydrocarbyl groups of various types. Indeed, silicone compounds have been extensively described in the art, see for instance US 4 076 648, US 4 021 365, US 4 749 740, US 4 983 316, EP 150 872, EP 217 501 and EP 499 364. The silicone compounds disclosed therein are suitable in the context of the present invention. Generally, the silicone compounds can be described as siloxanes having the general structure : 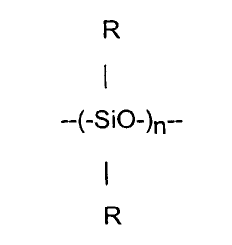 wherein n is from 20 to 2000, and where each R independently can be an alkyl or an aryl radical. Examples of such substituents are methyl, ethyl, propyl, isobutyl, and phenyl. Preferred polydiorganosiloxanes are polydimethylsiloxanes having trimethylsilyl end blocking units and having a viscosity at 25°C of from 5 x 10⁻⁵ m²/s to 0.1 m²/s, i.e. a value of n in the range 40 to 1500. These are preferred because of their ready availability and their relatively low cost.

A preferred type of silicone compounds useful for the present invention comprises a mixture of an alkylated siloxane of the type hereinabove disclosed and solid silica.

The solid silica can be a fumed silica, a precipitated silica or a silica made by the gel formation technique. The silica particles can be rendered hydrophobic by treating them with diakylsilyl groups and/or trialkylsilane groups either bonded directly onto the silica or by means of silicone resin. A preferred silicone compound comprises a hydrophobic silanated, most preferably trimethylsilanated silica having a particle size in the range from 10 mm to 20 mm and a specific surface area above 50 m²/g. Silicone compounds employed for the present invention suitably have an amount of silica in the range of 1 to 30% (more preferably 2.0 to 15%) by weight of the total weight of the silicone compounds resulting in silicone compounds having an average viscosity in the range of from 2 x 10⁻⁴m²/s to 1m²/s. Preferred silicone compounds may have a viscosity in the range of from 5 x 10⁻³m²/s to 0.1m²/s. Particularly suitable are silicone compounds with a viscosity of 2 x 10⁻²m²/s or 4.5 x 10⁻²m²/s.

Suitable silicone compounds for use herein are commercially available from various companies including Rhone Poulenc, Fueller and Dow Corning. Examples of silicone compounds for use herein are Silicone DB® 100 and Silicone Emulsion 2-3597® both commercially available from Dow Corning.

Another silicone compound is disclosed in Bartollota et al. U.S. Patent 3 933 672. Other particularly useful silicone compounds are the self-emulsifying silicone compounds, described in German Patent Application DTOS 2 646 126 published April 28, 1977. An example of such a compound is DC-544®, commercially available from Dow Corning, which is a siloxane-glycol copolymer.

Typically preferred silicone compounds are described in European Patent application EP-A-573699. Said compositions can comprise a silicone/silica mixture in combination with fumed nonporous silica such as Aerosil^{R}.

Fatty acids act synergistically as a foam reducing system with such a capped alkoxylated nonionic surfactant and/or silicone. Indeed, the reduction of foam height when measured in neat and diluted conditions (typically at a dilution level of 12.5 : 1000 (composition :water)) is faster and more important with some given peroxygen bleach-containing compositions comprising a surfactant (e.g. nonionic and/or zwitteionic surfactant) and a foam reducing system comprising a fatty acid together with capped alkoxylated nonionic surfactant and/or silicone), as compared to the reduction of foam height observed with the same composition but with only one of said foam reducing agent (either only fatty acid or only the capped alkoxylated nonionic surfactant or silicone) at the same total level of foam reducing agent.

More particularly, it has been found that the combination of a fatty acid with the capped alkoxylated nonionic surfactant performs dual functions when comprised by the present invention, said functions being not only to have a low foaming property but also to reduce the foaming of the surfactants herein both when the compositions herein are used in neat and diluted conditions.

Indeed, the combinations of foam reducing agents increase the collapse rate of the foam generated by the surfactants. As a result, when incorporated in the webs of the invention, a reduction of the generation of foam as well as an increase in the collapse rate is observed.

Optimum defoaming properties (consistency and strength of the suds) are obtained with the antifoaming agents comprising the fatty acid and the capped alkoxylated surfactant at a weight ratio of the fatty acid to the capped nonionic surfactant of 0.01 to 10, preferably from 0.1 to 5 and more preferably from 0.1 to 1. Also when the combination fatty acid and silicone is used as the foam reducing system herein, optimum defoaming properties (consistency and strenght of the suds) are obtained with the compositions comprising the fatty acid and the silicone at a weight ratio of the fatty acid to the silicone of 1·10⁴ to 1·10⁻¹, preferably from 1·10⁴ to 1 and more preferably from 1·10³ to 1·10¹.

In another preferred embodiment of the present invention, the three foam reducing agents described herein are used together to get optimum defoaming properties and are typically present at a weight ratio of fatty acid:capped nonionic surfactant: silicone of 1:1·10³:1·10⁻⁵ to 10:1:1 and preferably from 1: 1·10²:1·10⁻⁴ to 1:1:1.

Another advantage of the foam reducing system is that such capped nonionic surfactants, fatty acids and/or silicones are stable in presence of a peroxygen bleaching component.

A further advantage of the foam reducing system of the present invention is that they are physically and chemically stable upon prolonged periods of storage.

One preferred antifoaming agent in accordance with the present invention is available from Wacker as Wacker silicone antifoaming emulsion SE 2.

*Foaming* ("foam height" as used above) can be measured by using a Foammeter, a machine composed by 5 cylinders rotating around a horizontal axe, which can be filled with the product under evaluation.

Usually, the user sets the speed at 45 round per minute and the rotation time at 900 seconds. The test method may consists of 2 different steps related to different product concentrations:

### Foaming of neat product:

The Foam Meter cylinder is filled with the product under test until the first sign on the cylinder (about 500 grams of product) and the rotation is started.

### Foaming of diluted product:

Another cylinder is filled with tap water until the first sign on the cylinder (about 500 grams of tap water) and the appropriate quantity of test product depending on the dilution level under test is added before starting the rotation. As soon as the instrument stops, the foam height can be read on the scale outside each cylinder and recorded as the foam height at time 0. Then, the foam height for each cylinder can be recorded upon time, typically after 1, 2, 3, 4, 5, 10, 15, 20, 25, 30 minutes.

### Liquid composition

According to the present invention, the substrate material of the wipes is preferably impregnated or coated with a liquid composition. The composition may be aqueous, alcohol based or an emulsion, either a water-in-oil or a oil-in-water or a multiple emulsion, preferably the emulsion is a oil-in-water emulsion.

Typically, the composition will comprise from 0.1% to 50% by weight of said composition of actives and from 50% to 99.9% water, preferably de-ionised or distilled. Of the active component, preferably 2% to 20% are present in the oil phase and the remainder are present in the aqueous phase.

The liquid composition can provide a number of different benefits when released. For example, in wet-like cleaning wipes for perianal cleaning the water component is released and thereby provides the primary cleansing action for these wipes.

In a preferred embodiment of the present invention the liquid composition (preferably comprising water as a major constituent) comprises a disinfecting component comprising an antimicrobial compound, preferably an essential oil or an active thereof, and a bleach, preferably a peroxygen bleach. Disinfecting wipes comprising such a liquid composition provide effective disinfecting performance on a surface while being safe to the surface treated.

By "effective disinfecting performance" it is meant herein that the disinfecting wipes of the present invention allow significant reduction in the amount of bacteria on an infected surface. Indeed, effective disinfecting may be obtained on various micro-organisms including Gram positive bacteria like Staphylococcus aureus, and Gram negative bacteria like Pseudomonas aeruginosa, as well as on more resistant micro-organisms like fungi (e.g., Candida albicans) present on infected surfaces.

Another advantage of the disinfecting wipes according to the present invention is that besides the disinfecting properties delivered, good cleaning is also provided as the disinfecting polar phase may further comprise surfactants and/or solvents.

Such essential oils include, but are not limited to, those obtained from thyme, lemongrass, citrus, lemons, oranges, anise, clove, aniseed, cinnamon, geranium, roses, mint, lavender, citronella, eucalyptus, peppermint, camphor, sandalwood and cedar and mixtures thereof. Actives of essential oils to be used herein include, but are not limited to, thymol (present for example in thyme), eugenol (present for example in cinnamon and clove), menthol (present for example in mint), geraniol (present for example in geranium and rose), verbenone (present for example in vervain), eucalyptol and pinocarvone (present in eucalyptus), cedrol (present for example in cedar), anethol (present for example in anise), carvacrol, hinokitiol, berberine, terpineol, limonene, methyl salycilate and mixtures thereof. Preferred actives of essential oils to be used herein are thymol, eugenol, verbenone, eucalyptol, carvacrol, limonene and/or geraniol. Thymol may be commercially available for example from Aldrich, eugenol may be commercially available for example from Sigma, Systems - Bioindustries (SBI) - Manheimer Inc.

Typically, the antimicrobial compound or mixtures thereof will be present in the liquid composition at a level of from 0.001% to 5%, preferably from 0.001% to 3%, more preferably from 0.005% to 1%, by weight of liquid composition.

An important element of the internal disinfecting polar phase is a bleach or mixtures thereof. Any bleach known to those skilled in the art may be suitable to be used herein including any chlorine bleach as well as any peroxygen bleach. The presence of the bleach, preferably the peroxygen bleach, in the disinfecting wipes of the present invention contribute to the disinfecting properties of the wipes.

Suitable chlorine bleaches to be used herein include any compound capable of releasing chlorine when said compound is in contact with water. Suitable chlorine bleaches include alkali metal dichloroisocyanurates as well as alkali metal hypohalites like hypochlorite and/or hypobromite. Preferred chlorine bleaches are alkali metal hypochlorites. Various forms of alkali metal hypochlorite are commercially available, for instance sodium hypochlorite.

Preferred bleaches for use herein are peroxygen bleaches, more particularly hydrogen peroxide, or a water soluble source thereof, or mixtures thereof. Hydrogen peroxide is particularly preferred.

Peroxygen bleaches like hydrogen peroxide are preferred herein as they are generally well accepted from an environmental point of view. For example the decomposition products of hydrogen peroxide are oxygen and water.

As used herein, a hydrogen peroxide source refers to any compound which produces perhydroxyl ions when said compound is in contact with water. Suitable water-soluble sources of hydrogen peroxide for use herein include percarbonates, persilicates, persulphates such as monopersulfate, perborates, peroxyacids such as diperoxydodecandioic acid (DPDA), magnesium perphthalic acid, dialkylperoxides, diacylperoxides, performed percarboxylic acids, organic and inorganic peroxides and/or hydroperoxides and mixtures thereof.

Typically, the bleach or mixtures thereof is present at a level of from 0.001% to 15% by weight of the liquid composition, preferably from 0.001% to 5%, and more preferably from 0.005% to 2%.

The liquid composition may further comprise a detersive surfactant or a mixture thereof. Typically, the surfactant or mixtures thereof is present at a level of from 0.001% to 40% by weight of the total internal polar phase, preferably from 0.01 % to 10% and more preferably from 0.05% to 2%.

Suitable detersive surfactants to be used in the present invention include any surfactant known to those skilled in the art like nonionic, anionic, cationic, amphoteric and/or zwitterionic surfactants. Preferred detersive surfactants to be used herein are the amphoteric and/or zwitterionic surfactants.

Suitable amphoteric detersive surfactants to be used herein include amine oxides of the formula R¹R²R³NO, wherein each of R¹, R² and R³ is independently a saturated, substituted or unsubstituted, linear or branched hydrocarbon chain having from 1 to 30 carbon atoms. Preferred amine oxide surfactants to be used according to the present invention are amine oxides of the formula R¹R²R³NO, wherein R¹ is an hydrocarbon chain having from 1 to 30 carbon atoms, preferably from 6 to 20, more preferably from 8 to 16, most preferably from 8 to 12, and wherein R² and R³ are independently substituted or unsubstituted, linear or branched hydrocarbon chains having from 1 to 4 carbon atoms, preferably from 1 to 3 carbon atoms, and more preferably are methyl groups. R¹ may be a saturated, substituted or unsubstituted, linear or branched hydrocarbon chain. Suitable amine oxides for use herein are for instance natural blend C₈-C₁₀ amine oxides as well as C₁₂-C₁₆ amine oxides commercially available from Hoechst. Amine oxides are preferred herein as they deliver effective cleaning performance and further participate to the disinfecting properties of the disinfecting wipes herein.

Suitable zwitterionic surfactants to be used herein contain both cationic and anionic hydrophilic groups on the same molecule at a relatively wide range of pH's. The typical cationic group is a quaternary ammonium group, although other positively charged groups like phosphonium, imidazolinium and sulfonium groups can be used. The typical anionic hydrophilic groups are carboxylates and sulfonates, although other groups such as sulfates, phosphonates, and the like can be used. A generic formula for some zwitterionic surfactants to be used herein is

R¹-N+(R²)(R³)R⁴X-

wherein R¹ is a hydrophobic group; R² and R³ are each C₁-C₄ alkyl, hydroxy alkyl or other substituted alkyl group which can also be joined to form ring structures with the N; R⁴ is a moiety joining the cationic nitrogen atom to the hydrophilic group and is typically an alkylene, hydroxy alkylene, or polyalkoxy group containing from 1 to 10 carbon atoms; and X is the hydrophilic group which is preferably a carboxylate or sulfonate group. Preferred hydrophobic groups R¹ are alkyl groups containing from 1 to 24, preferably less than 18, more preferably less than 16 carbon atoms. The hydrophobic group can contain unsaturation and/or substituents and/or linking groups such as aryl groups, amido groups, ester groups and the like. In general, the simple alkyl groups are preferred for cost and stability reasons.

Highly preferred zwitterionic surfactants include betaine and sulphobetaine surfactants, derivatives thereof or mixtures thereof. Said betaine or sulphobetaine surfactants are preferred herein as they help disinfecting by increasing the permeability of the bacterial cell wall, thus allowing other active ingredients to enter the cell.

Furthermore, due to the mild action profile of said betaine or sulphobetaine surfactants, they are particularly suitable for the cleaning of delicate surfaces, e.g., hard surfaces in contact with food and/or babies. Betaine and sulphobetaine surfactants are also extremely mild to the skin and/or surfaces to be treated.

Suitable betaine and sulphobetaine surfactants to be used herein are the betaine/sulphobetaine and betaine-like detergents wherein the molecule contains both basic and acidic groups which form an inner salt giving the molecule both cationic and anionic hydrophilic groups over a broad range of pH values. Some common examples of these detergents are described in U.S. Pat. Nos. 2,082,275, 2,702,279 and 2,255,082, incorporated herein by reference. Preferred betaine and sulphobetaine surfactants herein are according to the formula 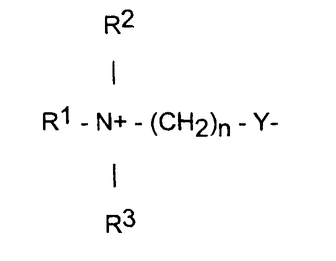 wherein R¹ is a hydrocarbon chain containing from 1 to 24 carbon atoms, preferably from 8 to 18, more preferably from 12 to 14, wherein R² and R³ are hydrocarbon chains containing from 1 to 3 carbon atoms, preferably 1 carbon atom, wherein n is an integer from 1 to 10, preferably from 1 to 6, more preferably is 1, Y is selected from the group consisting of carboxyl and sulfonyl radicals and wherein the sum of R¹, R² and R³ hydrocarbon chains is from 14 to 24 carbon atoms, or mixtures thereof.

Examples of particularly suitable betaine surfactants include C₁₂-C₁₈ alkyl dimethyl betaine such as coconut-betaine and C₁₀-C₁₆ alkyl dimethyl betaine such as laurylbetaine. Coconutbetaine is commercially available from Seppic under the trade name of Amonyl 265". Laurylbetaine is commercially available from Albright & Wilson under the trade name Empigen BB/L".

Other specific zwitterionic surfactants have the generic formulas:

R¹-C(O)-N(R²)-(C(R³)₂)ₙ-N(R²)₂⁽⁺⁾-(C(R³)₂)ₙ-SO₃⁽⁻⁾ ;

or

R¹-C(O)-N(R²)-(C(R³)₂)ₙ-N(R²)₂⁽⁺⁾-(C(R³)₂)ₙ-COO⁽⁻⁾

wherein each R¹ is a hydrocarbon, e.g. an alkyl group containing from 8 up to 20, preferably up to 18, more preferably up to 16 carbon atoms, each R² is either a hydrogen (when attached to the amido nitrogen), short chain alkyl or substituted alkyl containing from 1 to 4 carbon atoms, preferably groups selected from the group consisting of methyl, ethyl, propyl, hydroxy substituted ethyl or propyl and mixtures thereof, preferably methyl, each R³ is selected from the group consisting of hydrogen and hydroxy groups and each n is a number from 1 to 4, preferably from 2 to 3, more preferably 3, with no more than one hydroxy group in any (C(R³)₂) moiety. The R¹ groups can be branched and/or unsaturated. The R² groups can also be connected to form ring structures. A surfactant of this type is a C₁₀-C₁₄ fatty acylamidopropylene-(hydroxypropylene)sulfobetaine that is available from the Sherex Company under the trade name "Varion CAS sulfobetaine".

Suitable nonionic surfactants to be used herein are fatty alcohol ethoxylates and/or propoxylates which are commercially available with a variety of fatty alcohol chain lengths and a variety of ethoxylation degrees. Indeed, the HLB values of such alkoxylated nonionic surfactants depend essentially on the chain length of the fatty alcohol, the nature of the alkoxylation and the degree of alkoxylation. Surfactant catalogues are available which list a number of surfactants, including nonionics, together with their respective HLB values.

Particularly suitable for use herein as nonionic surfactants are the hydrophobic nonionic surfactants having an HLB (hydrophilic-lipophilic balance) below 16 and more preferably below 15. Those hydrophobic nonionic surfactants have been found to provide good grease cutting properties.

Preferred nonionic surfactants for use herein are nonionic surfactants according to the formula RO-(C₂H₄O)ₙ(C₃H₆O)ₘH, wherein R is a C₆ to C₂₂ alkyl chain or a C₆ to C₂₈ alkyl benzene chain, and wherein n+m is from 0 to 20 and n is from 0 to 15 and m is from 0 to 20, preferably n+m is from 1 to 15 and, n and m are from 0.5 to 15, more preferably n+m is from 1 to 10 and, n and m are from 0 to 10. The preferred R chains for use herein are the C₈ to C₂₂ alkyl chains. Accordingly, suitable hydrophobic nonionic surfactants for use herein are Dobanol R 91-2.5 (HLB= 8.1; R is a mixture of C₉ and C₁₁ alkyl chains, n is 2.5 and m is 0), or Lutensol R TO3 (HLB=8; R is a C₁₃ alkyl chains, n is 3 and m is 0), or Lutensol R AO3 (HLB=8; R is a mixture of C₁₃ and C₁₅ alkyl chains, n is 3 and m is 0), or Tergitol R 25L3 (HLB= 7.7; R is in the range of C₁₂ to C₁₅ alkyl chain length, n is 3 and m is 0), or Dobanol R 23-3 (HLB=8.1; R is a mixture of C₁₂ and C₁₃ alkyl chains, n is 3 and m is 0), or Dobanol R 23-2 (HLB=6.2; R is a mixture of C₁₂ and C₁₃ alkyl chains, n is 2 and m is 0), or Dobanol R 45-7 (HLB=11.6; R is a mixture of C₁₄ and C₁₅ alkyl chains, n is 7 and m is 0) Dobanol R 23-6.5 (HLB=11.9; R is a mixture of C₁₂ and C₁₃ alkyl chains, n is 6.5 and m is 0), or Dobanol R 25-7 (HLB=12; R is a mixture of C₁₂ and C₁₅ alkyl chains, n is 7 and m is 0), or Dobanol R 91-5 (HLB=11.6; R is a mixture of C₉ and C₁₁ alkyl chains, n is 5 and m is 0), or Dobanol R 91-6 (HLB=12.5; R is a mixture of C₉ and C₁₁ alkyl chains, n is 6 and m is 0), or Dobanol R 91-8 (HLB=13.7; R is a mixture of C₉ and C₁₁ alkyl chains, n is 8 and m is 0), Dobanol R 91-10 (HLB=14.2; R is a mixture of C₉ to C₁₁ alkyl chains, n is 10 and m is 0), or mixtures thereof. Preferred herein are Dobanol R 91-2.5, or Lutensol R TO3, or Lutensol R AO3, or Tergitol R 25L3, or Dobanol R 23-3, or Dobanol R 23-2, or Dobanol R 23-10, or mixtures thereof. DobanolR surfactants are commercially available from SHELL. LutensolR surfactants are commercially available from BASF and the Tergitol R surfactants are commercially available from UNION CARBIDE.

Suitable anionic surfactants to be used herein include water soluble salts or acids of the formula ROSO₃M wherein R is preferably a C₆-C₂₄ hydrocarbyl, preferably an alkyl or hydroxyalkyl having a C₈-C₂₀ alkyl component, more preferably a C₈-C₁₈ alkyl or hydroxyalkyl, and M is H or a cation, e.g., an alkali metal cation (e.g., sodium, potassium, lithium), or ammonium or substituted ammonium (e.g., methyl-, dimethyl-, and trimethyl ammonium cations and quaternary ammonium cations, such as tetramethyl-ammonium and dimethyl piperdinium cations and quaternary ammonium cations derived from alkylamines such as ethylamine, diethylamine, triethylamine, and mixtures thereof, and the like).

Other suitable anionic surfactants to be used herein include alkyl-diphenylether-sulphonates and alkyl-carboxylates. Other anionic surfactants can include salts (including, for example, sodium, potassium, ammonium, and substituted ammonium salts such as mono-, di- and triethanolamine salts) of soap, C₉-C₂₀ linear alkylbenzenesulfonates, C₈-C₂₂ primary or secondary alkanesulfonates, C₈-C₂₄ olefinsulfonates, sulfonated polycarboxylic acids prepared by sulfonation of the pyrolyzed product of alkaline earth metal citrates, e.g., as described in British patent specification No. 1,082,179, C₈-C₂₄ alkylpolyglycolethersulfates (containing up to 10 moles of ethylene oxide); alkyl ester sulfonates such as C₁₄₋₁₆ methyl ester sulfonates; acyl glycerol sulfonates, fatty oleyl glycerol sulfates, alkyl phenol ethylene oxide ether sulfates, paraffin sulfonates, alkyl phosphates, isethionates such as the acyl isethionates, N-acyl taurates, alkyl succinamates and sulfosuccinates, monoesters of sulfosuccinate (especially saturated and unsaturated C₁₂-C₁₈ monoesters) diesters of sulfosuccinate (especially saturated and unsaturated C₆-C₁₄ diesters), acyl sarcosinates, sulfates of alkylpolysaccharides such as the sulfates of alkylpolyglucoside (the nonionic nonsulfated compounds being described below), branched primary alkyl sulfates, alkyl polyethoxy carboxylates such as those of the formula RO(CH₂CH₂O)ₖCH₂COO-M+ wherein R is a C₈-C₂₂ alkyl, k is an integer from 0 to 10, and M is a soluble salt-forming cation. Resin acids and hydrogenated resin acids are also suitable, such as rosin, hydrogenated rosin, and resin acids and hydrogenated resin acids present in or derived from tall oil. Further examples are given in "Surface Active Agents and Detergents" (Vol. I and II by Schwartz, Perry and Berch). A variety of such surfactants are also generally disclosed in U.S. Patent 3,929,678, issued December 30, 1975 to Laughlin, et al. at Column 23, line 58 through Column 29, line 23 (herein incorporated by reference).

Preferred anionic surfactants for use herein are the alkyl benzene sulfonates, alkyl sulfates, alkyl alkoxylated sulfates, paraffin sulfonates and mixtures thereof.

The internal disinfecting polar phase according to the present invention has a pH of from 1 to 12, preferably from 1.5 to 10, and more preferably from 2 to 9. The pH can be adjusted by using alkalinizing agents or acidifying agents. Examples of alkalinizing agents are alkali metal hydroxides, such as potassium and/or sodium hydroxide, or alkali metal oxides such as sodium and/or potassium oxide. Examples of acidifying agents are organic or inorganic acids such as citric or sulfuric acid.

Solvents may be present in the liquid compositionf. These solvents will, advantageously, give an enhanced cleaning to the disinfecting wipes of the present invention. Suitable solvents for incorporation herein include propylene glycol derivatives such as n-butoxypropanol or n-butoxypropoxypropanol, water-soluble CARBITOL, solvents or water-soluble CELLOSOLVE, solvents. Water-soluble CARBITOL, solvents are compounds of the 2-(2-alkoxyethoxy)ethanol class wherein the alkoxy group is derived from ethyl, propyl or butyl. A preferred water-soluble carbitol is 2-(2-butoxyethoxy)ethanol also known as butyl carbitol. Water-soluble CELLOSOLVE, solvents are compounds of the 2-alkoxyethoxyethanol class, with 2-butoxyethoxyethanol being preferred. Other suitable solvents are benzyl alcohol, methanol, ethanol, isopropyl alcohol and diols such as 2-ethyl-1,3-hexanediol and 2,2,4-trimethyl-1,3-pentanediol and mixture thereof. Preferred solvents for use herein are n-butoxypropoxypropanol, butyl carbitol, and mixtures thereof. A most preferred solvent for use herein is butyl carbitol.

The liquid composition may further comprise other optional ingredients including radical scavengers, chelating agents, thickeners, builders, buffers, stabilizers, bleach activators, soil suspenders, dye transfer agents, brighteners, anti dusting agents, enzymes, dispersant, dye transfer inhibitors, pigments, perfumes, and dyes and the like.

Suitable radical scavengers for use herein include the well-known substituted mono and di hydroxy benzenes and derivatives thereof, alkyl- and aryl carboxylates and mixtures thereof. Preferred radical scavengers for use herein include di-tert-butyl hydroxy toluene (BHT), p-hydroxy-toluene, hydroquinone (HQ), di-tert-butyl hydroquinone (DTBHQ), mono-tert-butyl hydroquinone (MTBHQ), tert-butyl-hydroxy anysole, p-hydroxy-anysol, benzoic acid, 2,5-dihydroxy benzoic acid, 2,5-dihydroxyterephtalic acid, toluic acid, catechol, t-butyl catechol, 4-allyl-catechol, 4-acetyl catechol, 2-methoxy-phenol, 2-ethoxy-phenol, 2-methoxy-4-(2-propenyl)phenol, 3,4-dihydroxy benzaldehyde, 2,3-dihydroxy benzaldehyde, benzylamine, 1,1,3-tris(2-methyl-4-hydroxy-5-t-butylphenyl) butane, tert-butyl-hydroxy-anyline, p-hydroxy anyline as well as n-propyl-gallate. Highly preferred for use herein is di-tert-butyl hydroxy toluene, which is for example commercially available from SHELL under the trade name IONOL CP".

Typically, the radical scavenger, or a mixture thereof, is present in the liquid composition up to a level of 5% by weight, preferably from 0.001% to 3% by weight, and more preferably from 0.001% to 1.5%.

Suitable chelating agents to be used herein may be any chelating agent known to those skilled in the art such as the ones selected from the group consisting of phosphonate chelating agents, amino carboxylate chelating agents or other carboxylate chelating agents, or polyfunctionally-substituted aromatic chelating agents and mixtures thereof.

Such phosphonate chelating agents may include etidronic acid (1-hydroxyethylidene-bisphosphonic acid or HEDP) as well as amino phosphonate compounds, including amino alkylene poly (alkylene phosphonate), alkali metal ethane 1-hydroxy diphosphonates, nitrilo trimethylene phosphonates, ethylene diamine tetra methylene phosphonates, and diethylene triamine penta methylene phosphonates. The phosphonate compounds may be present either in their acid form or as salts of different cations on some or all of their acid functionalities. Preferred phosphonate chelating agents to be used herein are diethylene triamine penta methylene phosphonates. Such phosphonate chelating agents are commercially available from Monsanto under the trade name DEQUEST.

Polyfunctionally-substituted aromatic chelating agents may also be useful herein. See U.S. Patent 3,812,044, issued May 21, 1974, to Connor et al. Preferred compounds of this type in acid form are dihydroxydisulfobenzenes such as 1,2-dihydroxy -3,5-disulfobenzene.

A preferred biodegradable chelating agent for use herein is ethylene diamine N,N'- disuccinic acid, or alkali metal, or alkaline earth, ammonium or substitutes ammonium salts thereof or mixtures thereof. Ethylenediamine N,N'- disuccinic acids, especially the (S,S) isomer have been extensively described in US patent 4, 704, 233, November 3, 1987 to Hartman and Perkins. Ethylenediamine N,N'-disuccinic acid is, for instance, commercially available under the tradename ssEDDS, from Palmer Research Laboratories.

Suitable amino carboxylate chelating agents useful herein include ethylene diamine tetra acetate, diethylene triamine pentaacetate, diethylene triamine pentaacetate (DTPA), N-hydroxyethylethylenediamine triacetate, nitrilotri-acetate, ethylenediamine tetraproprionate, triethylenetetraaminehexa-acetate, ethanoldiglycine, propylene diamine tetracetic acid (PDTA) and methyl glycine di-acetic acid (MGDA), both in their acid form, or in their alkali metal, ammonium, and substituted ammonium salt forms. Particularly suitable to be used herein are diethylene triamine penta acetic acid (DTPA), propylene diamine tetracetic acid (PDTA) which is, for instance, commercially available from BASF under the trade name Trilon FS, and methyl glycine di-acetic acid (MGDA).

Further carboxylate chelating agents to be used herein includes malonic acid, salicylic acid, glycine, aspartic acid, glutamic acid, dipicolinic acid and derivatives thereof, or mixtures thereof.

Typically, the chelating agent, or a mixture thereof, is present in the liquid composition at a level of from 0.001% to 5% by weight, preferably from 0.001% to 3% by weight and more preferably from 0.001% to 1.5%.

Other water-soluble or dispersible materials that can be present in the liquid composition include thickeners and viscosity modifiers. Suitable thickeners and viscosity modifiers include polyacrylic and hydrophobically modified polyacrylic resins such as Carbopol and Pemulen, starches such as corn starch, potato starch, tapioca, gums such as guar gum, gum arabic, cellulose ethers such as hydroxypropyl cellulose, hydroxyethyl cellulose, carboxymethyl cellulose, and the like. These thickeners and viscosity modifiers will typically be included in a concentration in the range of from about 0.05 to about 0.5% of the liquid composition.

Again, where water is a major constituent of the liquid composition, water-soluble or dispersible materials that can be present in the internal phase include polycationic polymers to provide steric stabilization at the polar phase-lipid phase interface and nonionic polymers that also stabilize the emulsion. Suitable polycationic polymers include Reten 201, Kymene® 557H and Acco 711. Suitable nonionic polymers include polyethylene glycols (PEG) such as Carbowax and poly(propylene glycol) butyl ether. These polycationic and nonionic polymers will typically be included in a concentration in the range of from about 0.05 to about 1.0% of the liquid composition.

According to the present invention the wet wipes are preferably provided with an emulsion composition comprising a oil phase in the range of 1% to 20%, preferably 2% to 10%, by weight of the composition. Advantageously, the oil based phase is derived from natural resources such as from vegetable or animal oils or may be synthetic or any mixtures thereof. Suitable vegetable and animal oils for use herein include waxes such as beeswax, lanolin, candelilla, and oils such as glycerine esters and glycerine ethers, fatty acid alcohols, fatty acid esters and fatty acid ethers such as caprylic and capric triglycerides and octylpalmitate. Suitable mineral oils include petroleum based oils such as paraffin and petroleum jelly. Synthetic oils for use herein include ethylenic polymers for example polyethylene wax or silicone based oils. Suitable silicon oils include polydimethylsiloxanes, volatile cyclomethicones, dimethiconols, siloxysilicates and amino- and phenyl derivatives of siloxanes and mixtures thereof. Examples include dimethicone (Dow Corning 200 Fluids), cyclomethicone and dimethiconol (Dow Corning 1401 Fluid), cetyl dimethicone (Dow Corning 2502 Fluid), dimethicone and trimethylsiloxysilicate (Dow Corning 593 Fluid), cyclomethicone (Dow Coming 244, 245, 344 or 345 Fluid), phenyl trimethicone (Dow Coming 556 Fluid), or combinations thereof.

The oil-in-water emulsions typically require emulsifying agents. The emulsifying agents which may be used in the present invention are preferably capable of primary emulsification of oil-in-water emulsions. The emulsifying agent is present in the range of 0.02% to 5.0%, preferably 0.02% to 3.0%, by weight of the composition.

In a preferred embodiment the emulsifying agent is a polymeric type of emulsifying agent such as a copolymer of C10-C30 alkyl acrylates and one or more monomers of acrylic acid, methylacrylic acid or one of their simple esters cross linked with an allyl ether of sucrose or an allyl ether of pentaerythritol. The emulsifying agents which are thus useful in the present invention include Ceteareth-12, Ceteareth-20 or Pemulen TR1 and TR2 which are available from B.F. Goodrich company of the USA. However, other known emulsifying agents such as ethoxylated fatty alcohols, glycerine esters of fatty acids, soaps, sugar derived agents are also suitable for use herein. Other useful emulsifying agents include those disclosed in detail in EP-A-328 355.

According to the present invention the composition may comprise a stability agent or preservative. Stability agents suitable for use herein include phenoxyethanol preferably present in the range of from 0.1 to 1.0%, sodium benzoate, potassium sorbate, methylparaben, propylparaben, ethylparaben, butylparaben, sodium benzoate, potassium sorbate, benzalkonium chloride, and disodium salt ethylenediamine tetraacetic acid (hereinafter referred to as EDTA) or other EDTA salts (sequestrenes). Sequestrene is a series of complexing agents and metal complexes general of ethylenediaminetetraacetic acid and salts. The total quantity of stability agents should be in the range of 0.1% to 4.0% by weight of the composition.

The composition of the present invention may further comprise from 0.02% to 5.0% by weight of said composition of an emollient or moisturiser. Preferably the emollient is water soluble and includes polyhydric alcohols, such as propylene glycol, glycerin, and also water soluble lanolin derivatives.

The composition of the present invention may have any pH value between 0 and 14 depending on the use of the wet wipes envisaged. For some applications a liquid composition with pH value below 7 is preferred, while for other applications a liquid composition with pH value above 7 is preferred.

In preparing wet wipe products according to the present invention, the composition is applied to at least one surface of the substrate material. The composition can be applied at any time during the manufacture of the wet wipe. Preferably the composition can be applied to the substrate after the substrate has been dried. Any variety of application methods that evenly distribute lubricious materials having a molten or liquid consistency can be used. Suitable methods include spraying, printing, (e.g. flexographic printing), coating (e.g. gravure coating or flood coating) extrusion whereby the composition is forced through tubes in contact with the substrate whilst the substrate passes across the tube or combinations of these application techniques. For example spraying the composition on a rotating surface such as calender roll that then transfers the composition to the surface of the substrate. The composition can be applied either to one surface of the substrate or both surfaces, preferably both surfaces. The preferred application method is extrusion coating.

The composition can also be applied uniformly or non uniformly to the surfaces of the substrate. By non uniform it is meant that for example the amount, pattern of distribution of the composition can vary over the surface of the substrate. For example some of the surface of the substrate can have greater or lesser amounts of composition, including portions of the surface that do not have any composition on it. Preferably however the composition is uniformly applied to the surfaces of the wipes. The composition is typically applied in an amount of from about 0.5 g to 10 g per gram of substrate, preferably from 1.0 g to 5 g per gram of substrate, most preferably from 2 g to 4 g per gram of dry substrate.

Preferably, the composition can be applied to the substrate at any point after it has been dried. For example the composition can be applied to the substrate prior to calendering or after calendering and prior to being wound up onto a parent roll. Typically, the application will be carried out on a substrate unwound from a roll having a width equal to a substantial number of wipes it is intended to produce. The substrate with the composition applied thereto is then subsequently perforated utilizing standard techniques in order to produce the desired perforation line.

### Disinfecting agent

According to the present invention the web preferably also comprises an disinfecting agent. Preferably the disinfecting agent is comprising in the liquid composition described hereinabove. The disinfecting agent is typically selected from the group consisting of an essential oil and an active thereof, paraben (e.g., methyl paraben, ethyl paraben), glutaraldehyde and mixtures thereof. Essential oils or actives thereof are the preferred antimicrobial compounds to be used herein.

Suitable essential oils or actives thereof to be used herein are those essential oils which exhibit antimicrobial activity and more particularly antibacterial activity. By "actives of essential oils" it is meant herein any ingredient of essential oils that exhibits antimicrobial/antibacterial activity. A further advantage of said essential oils and actives hereof is that they impart pleasant odour to the disinfecting wipes according to the present invention without the need of adding a perfume. Indeed, the disinfecting wipes according to the present invention deliver not only excellent disinfecting performance on infected surfaces but also good scent.

Such essential oils include, but are not limited to, those obtained from thyme, lemongrass, citrus, lemons, oranges, anise, clove, aniseed, cinnamon, geranium, roses, mint, lavender, citronella, eucalyptus, peppermint, camphor, sandalwood and cedar and mixtures thereof. Actives of essential oils to be used herein include, but are not limited to, thymol (present for example in thyme), eugenol (present for example in cinnamon and clove), menthol (present for example in mint), geraniol (present for example in geranium and rose), verbenone (present for example in vervain), eucalyptol and pinocarvone (present in eucalyptus), cedrol (present for example in cedar), anethol (present for example in anise), carvacrol, hinokitiol, berberine, terpineol, limonene, methyl salycilate and mixtures thereof. Preferred actives of essential oils to be used herein are thymol, eugenol, verbenone, eucalyptol, carvacrol, limonene and/or geraniol. Among these actives thymol, geraniol, methyl salycilate and mixtures thereof are preferred. Thymol may be commercially available for example from Aldrich, eugenol may be commercially available for example from Sigma, Systems - Bioindustries (SBI) - Manheimer Inc.

According to the present invention the disinfecting agent is preferably present in the liquid composition at a level of from 0.001% to 5%, preferably from 0.001% to 3%, more preferably from 0.005% to 1%, yet more preferably from 0.01% to 0.1% by weight of liquid composition.

It is recognised that any bleach which is preferably present in the liquid composition contributes to the disinfecting properties of the wet wipes. The term disinfecting agent, as used herein, however does not encompass bleaches.

### Shine Test: Methods and Results

Shine performance is evaluated on six equal flat stainless steel surfaces as usually present in the house. A test wet wipe and a reference wet wipe are used.

The stainless steel surfaces are each rubbed with one wet wipe (either the test wet wipe or the reference wet wipe) our times in horizontal direction (left/right) and four times in vertical direction (up/down). Three stainless steel surfaces are treated with the test wet wipe, and three stainless steel surface are treated with the reference wet wipe .The stainless steel surfaces are let to dry for ten minutes before grading by expert judges.

A panel of judges is then asked to evaluate the treated stainless steel surfaces for amount of filming/streaking. A numerical value describing the amount of filming/streaking is assigned to each wipe. For the test results, a -4/+4 scale is used:
-4 = there is a huge difference between the tested product and the reference. The tested product is worse than the reference
0 = there is no difference between the tested product and the reference.
+4 = there is a huge difference between the tested product and the reference. The tested product is better than the reference

The following table summarises the test results with a test wet wipe and a reference wet wipe each loaded with 3.25 g of the liquid composition specified in Example 1. The test wet wipe comprises a substrate made by a method according to the present invention and comprising the compounds given in Example 1. The reference wet wipe comprises the same compounds, but the antifoaming agent is not comprises by the substrate but by the liquid composition.

| REFERENCE WET WIPE | TEST WET WIPE |
|---|---|
| Wipe with 0.4% silicone antifoaming agent added to liquid composition for impregnation therewith | Wipe with 0.4% silicone antifoaming agent incorporated in the substrate according to the invention |
| (Reference for rating) | Rating: + 2 PSU (less streakes left on surfaces, better shine) |

### EXAMPLE I

A) Substrate
The substrate is a air laid nonwoven substrate comprising 70% pulp, 15% PET and 15% Rowene™ SB 5550 binder. The substrate further comprises 0.4% Wacker silicone antifoaming emulsion SE 2 as an antifoaming agent. The substrate has a basis weight of 73g/m².
B) Liquid composition Preparation
A liquid composition is prepared from the ingredients shown in Table I.

**Table I**

| Ingredients: | Percentage |
|---|---|
| Distilled Water | 87 |
| Salicylic acid | 0.03 |
| Hydrogen Peroxide | 1.0 |
| Ethanol | 9.4 |
| C-12 Amine Oxide | 0.4 |
| Geraniol | 0.04 |
| Thymol | 0.025 |
| Citric acid | 0.75 |
| Glycol butyl ether | 1.4 |
| | |

The liquid composition has a pH value of 2.9.
C) Applying the liquid composition to the Substrate
The liquid composition prepared in step B is applied to the substrate. 3.25g of lotion per gram of substrate material is applied to the substrate.

The coated substrate is then severed to form individual wipes, folded and stacked. The stack is then sealed to yield finished product wipe.

## Claims

1. A process for producing a web, said process comprising in any sequence the following steps:
step a) the application of a binder followed by the curing of said binder
step b) the application of an antifoaming agent followed by the curing of said antifoaming agent

2. A process according to Claim 1 wherein said step a) is followed by said step b).

3. A process according to any one of the preceding claims wherein said step a) is immediately followed by said step b).

4. A process according to any one of the preceding claims wherein said antifoaming agent comprises silicone.

5. A process according to any one of the preceding claims comprising an additional step c) wherein a liquid composition is applied to said web.

6. A process according to Claim 5 wherein step c) occurs after steps a) and b).

7. A process according to any one of the preceding claims wherein said web is a non-woven web.

8. A process according to Claim 7 wherein said web is an air-laid web.

9. A process according to any one of the preceding claims wherein said web comprises a latex binder, a non-acrylate binder or a mixture thereof.

10. A process according to Claim 9 wherein said web comprises from 5% to 30 % of said binders.
